# EUROPEAN PATENT SPECIFICATION

(11) **EP 3 810 200 B1**
(45) Date of publication and mention of the grant of the patent: **14.08.2024**
(21) Application number: 19730817.4
(22) Date of filing: 19.06.2019
(51) Int. Cl.: A61K 31/437, A61K 31/4523, A61K 31/506, A61K 45/06, A61K 31/519, A61P 35/00

(54) **COMPOSITIONS FOR TREATING MELANOMA**
ZUSAMMENSETZUNGEN ZUR BEHANDLUNG VON MELANOM
COMPOSITIONS PERMETTANT DE TRAITER LE MÉLANOME

(30) Priority: 20.06.2018 EP 18305776
(43) Date of publication of application: 28.04.2021
(73) Proprietor: INSERM (Institut National de la Santé et de la Recherche Médicale), 75013 Paris (FR); Université Côte d'Azur, 06103 Nice (FR)
(72) Inventor: TARTARE - DECKERT, Sophie, 06204 NICE CEDEX3 (FR); DECKERT, Marcel, 06204 NICE CEDEX 3 (FR); BERESTJUK, Ilona, 06204 NICE CEDEX 3 (FR)
(74) Representative: Inserm Transfert
(86) International application number: PCT/EP2019/066224
(87) International publication number: WO 2019/243431

(56) References cited:
- WO-A2-2014/105966
- KAVITHA GOWRISHANKAR ET AL: "Acquired Resistance to BRAF Inhibition Can Confer Cross-Resistance to Combined BRAF/MEK Inhibition", JOURNAL OF INVESTIGATIVE DERMATOLOGY, vol. 132, no. 7, 1 July 2012 (2012-07-01), NL, pages 1850 - 1859, XP055530651, ISSN: 0022-202X, DOI: 10.1038/jid.2012.63
- FRANCESCO SABBATINO ET AL: "PDGFR[alpha] up-regulation mediated by sonic hedgehog pathway activation leads to BRAF inhibitor resistance in melanoma cells with BRAF mutation", ONCOTARGET, 15 April 2014 (2014-04-15), United States, pages 1926 - 1941, XP055530735, Retrieved from the Internet <URL:https://www.ncbi.nlm.nih.gov/pmc/articles/PMC4039118/pdf/oncotarget-05-1926.pdf> DOI: 10.18632/oncotarget.1878
- ROSKOSKI ROBERT ED - ACOSTA GABRIELA ET AL: "Allosteric MEK1/2 inhibitors including cobimetanib and trametinib in the treatment of cutaneous melanomas", PHARMACOLOGICAL RESEARCH, ACADEMIC PRESS, LONDON, GB, vol. 117, 9 December 2016 (2016-12-09), pages 20 - 31, XP029921264, ISSN: 1043-6618, DOI: 10.1016/J.PHRS.2016.12.009

## Description

### FIELD OF THE INVENTION:

The invention is in the field of oncology. More particularly, the invention relates to methods and compositions to treat melanoma.

### BACKGROUND OF THE INVENTION:

One of the hallmarks of cancer cells is their exquisite ability to adapt to micro-environmental influences such as the nature of the stroma including the extracellular matrix (ECM) and therapeutic insults. This is particularly true for malignant melanoma, which is one of the most virulent cancers and refractory diseases. Approximately 50% of skin melanomas carry activating mutations in the BRAF oncogene, leading to activation of the mitogen-activated protein kinase (MAPK) pathway. Inhibition of the BRAF oncoprotein by targeted drugs has markedly improved the clinical outcome of patients with BRAFV600E/K mutant melanoma. The probability to achieve an objective response with combined inhibition of both BRAF and the downstream MEK in these patients is around 70%. However, after one year,, half of the patient develops secondary resistance. Acquired resistance to targeted drugs mostly involved restoration of the MAPK signaling pathway through mutations, amplifications or reprogramming of key intracellular regulators of the MAPK pathway. However, in addition to mechanisms of resistance intrinsic to the cancer cell, there exist dynamic, de novo mechanisms orchestrated by the tumor microenvironment resulting in an environmental-mediated drug resistance (EM-DR). Understanding and further identifying mechanisms of EM-DR in melanoma may thus help to develop more effective therapeutic strategies to delay or prevent the emergence of therapy-resistant clones.

Cancer cells are embedded in a complex ECM with unique biochemical and biophysical properties that can have a dramatic impact on tumor cell behavior and disease progression. The ECM is a dynamic network of macromolecules that plays a key role in establishing tumor niches. The ECM is primarily composed of fibrillar and non-fibrillar collagens, hyaluronic acid, proteoglycans, and adhesive glycoproteins (laminin, fibronectin, thrombospondins, vitronectin...) providing a structural framework for tissues and tumors. The ECM also contains matrix-remodeling enzymes and acts as a reservoir for cytokines and growth factors. Cancer-associated fibroblasts (CAFs) are the major producers of the tumorigenic ECM and constitute the main stromal components in many types of malignancies. CAFs are a heterogeneous population of activated and differentiated fibroblasts that are mainly derived from normal tissue resident fibroblast cells. Emerging studies have demonstrated that many of the tumor-supporting, immunosuppressive and therapy-resisting properties of the stroma can be attributed to the activity of CAFs. In melanoma, studies have shown that stromal cells including melanoma-associated fibroblasts (MAF) secrete various environmental factors that enable melanoma cells to evade and tolerate BRAF inhibitor therapies. These extracellular-derived signals include cytokines, growth and inflammatory factors, as well as ECM components such as fibronectin. Adhesion of tumor cells to ECM is one of the components of EM-DR. However, the influence of matrix-mediated drug resistance (MM-DR) in response to targeted therapies and the nature of ECM receptors that drives the MM-DR phenotype in melanoma have not been addressed in detail.

Kavitha Gowrishankar et al. discloses that acquired resistance to BRAF inhibition can confer cross-resistance to combined BRAF/MEK inhibition (Gowrishankar, Kavitha, et al. "Acquired resistance to BRAF inhibition can confer cross-resistance to combined BRAF/MEK inhibition." Journal of investigative dermatology 132.7 (2012): 1850-1859).

### SUMMARY OF THE INVENTION:

The references to the methods of treatment by therapy or surgery or in vivo diagnosis methods of this description are to be interpreted as references to compounds, pharmaceutical compositions and medicaments of the present invention for use in those methods.

The present invention is defined by the claims. Any subject-matter falling outside the scope of the claims is provided for information only. The invention relates to a therapeutically effective amount of: i) vemurafenib, an inhibitor of BRAF, ii) trametinib, an inhibitor of MEK and iii) imatinib, an inhibitor of DDR1/2 for use in the treatment of melanoma in a subject in need thereof. The invention also relates to a pharmaceutical composition comprising i) the vemurafenib, inhibitor of BRAF, ii) the trametinib, inhibitor of MEK and iii) the imatinib, inhibitor of DDR1/2. Further, the invention relates to the use of said composition in therapy, in particular in the treatment of melanoma.

### DETAILED DESCRIPTION OF THE INVENTION:

Targeted therapies against the BRAF oncogenic pathway have achieved limited clinical benefit to date in patients with melanoma due to development of acquired drug resistance. While the role of the tumor microenvironment in resistance to targeted therapies is well accepted, little is known about contributions of the matrix itself and melanoma cell surface molecules involved. To model the contribution of the ECM in melanoma cell responsiveness to BRAF and MEK inhibition, inventors generated fibroblast-derived 3D ECM from fibroblasts isolated from patient-derived biopsies (melanoma-associated fibroblasts, MAF) and analyzed the process of matrix-mediated drug resistance (MM-DR). Here, they find that adhesion of melanoma cells to fibroblast-derived ECM abrogates anti-proliferative responses to combination therapy with BRAF inhibitor (Vemurafenib) and MEK inhibitor (Trametinib) by maintaining phosphorylation of Rb, cell cycle progression, and expression of the pro-survival protein Survivin. This MM-DR is dependent on tyrosine kinase receptors DDR1 and DDR2, two receptors for collagens. Genetic depletion of DDRs or their pharmacological inhibition overcomes MM-DR and sensitizes melanoma cells to dual BRAF and MEK inhibitor therapy leading to reduced Rb phosphorylation, the cleavage of the apoptosis marker Caspase 3 and melanoma cell death. In melanoma cell-derived xenograft models, targeting DDR1 and DDR2 by Imatinib enhances BRAF inhibitor anti-tumor efficacy, delays the onset of acquired resistance and counteracts targeted therapy-induced tumor fibrosis. Their findings reveal a novel role of DDRs in drug resistance and provide a rationale to inhibit DDRs signaling to disrupt the therapy-resisting properties of the matrix microenvironment on melanoma cells. Targeting DDRs with Imatinib or Nilotinib, two clinically approved drugs for leukemia may thus provide a novel strategy to prevent the emergence of drug resistance in melanoma patients on targeted therapies.

Accordingly, the invention relates to a method for treating melanoma in a subject in need thereof comprising a step of administering said subject with a therapeutically effective amount of: i) vemurafenib, an inhibitor of BRAF, ii) trametinib, an inhibitor of MEK and iii) imatinib, an inhibitor of DDR1/2.

In a further embodiment, the invention relates to i) vemurafenib, an inhibitor of BRAF, ii) trametinib, an inhibitor of MEK and iii) imatinib, an inhibitor of DDR1/2 as a combined preparation for simultaneous, separate or sequential use in the treatment of melanoma.

In one embodiment, the inhibitor of BRAF, the inhibitor of MEK and the inhibitor of DDR1/2 of the invention are administered in combination with a classical treatment of melanoma.

In particular embodiment, the inhibitor of BRAF, the inhibitor of MEK and the inhibitor of DDR1/2 of the invention are administered in combination with an anti-PD-1 antibody.

As used herein, the terms "treating" or "treatment" refer to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "melanoma" also known as malignant melanoma, refers to a type of cancer that develops from the pigment-containing cells, called melanocytes. There are three general categories of melanoma: 1) cutaneous melanoma which corresponds to melanoma of the skin; it is the most common type of melanoma; 2) mucosal melanoma which can occur in any mucous membrane of the body, including the nasal passages, the throat, the vagina, the anus, or in the mouth; and 3) ocular melanoma also known as uveal melanoma or choroidal melanoma, is a rare form of melanoma that occurs in the eye. In a particular embodiment, the melanoma is cutaneous melanoma.

As used herein, the term "subject" denotes a mammal, such as a rodent, a feline, a canine, and a primate. Particularly, the subject according to the invention is a human. More particularly, the subject according to the invention has or is susceptible to have melanoma. In particular embodiment, the subject has or is susceptible to have cutaneous melanoma. In a particular embodiment, the subject has or is susceptible to have melanoma resistant. In a particular embodiment, the subject has or is susceptible to have BRAFV600E/K mutation.

As used herein, the term "BRAF" is a member of the Raf kinase family of serine/threonine-specific protein kinases. This protein plays a role in regulating the MAP kinase / ERKs signaling pathway, which affects cell division, differentiation, and survival. A number of mutations in BRAF are known. In particular, the V600E mutation is prominent. Other mutations which have been found are R461I, I462S, G463E, G463V, G465A, G465E, G465V, G468A, G468E, N580S, E585K, D593V, F594L, G595R, L596V, T598I, V599D, V599E, V599K, V599R, K600E, A727V, and most of these mutations are clustered to two regions: the glycine-rich P loop of the N lobe and the activation segment and flanking regions. In a particular embodiment, the BRAF mutation is V600E/K in the context of the invention. As used herein, the term "inhibitor of BRAF" refers to a natural or synthetic compound that has a biological effect to inhibit the activity or the expression of BRAF. More particularly, such compound by inhibiting BRAF activity reduces cell division, differentiation, and secretion. In a particular embodiment, the inhibitor of BRAF is a peptide, petptidomimetic, small organic molecule, antibody, aptamers, siRNA or antisense oligonucleotide. The term "peptidomimetic" refers to a small protein-like chain designed to mimic a peptide. In a particular embodiment, the inhibitor of BRAF is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity.

In the present invention, the inhibitor of BRAF is Vemurafenib. Vemurafenib also known as PLX4032, RG7204 ou RO5185426 and commercialized by Roche as zelboraf.

As used herein, the term "MEK" refers to Mitogen-activated protein kinase, also known as MAP2K, MEK, MAPKK. It is a kinase enzyme, which phosphorylates mitogen-activated protein kinase (MAPK). As used herein, the term "inhibitor of MEK" refers to a natural or synthetic compound that has a biological effect to inhibit the activity or the expression of MEK. More particularly, such compound by inhibiting BRAF activity reduces phosphorylation of MAPK.

In the present invention, the inhibitor of MEK is Trametinib also known as mekinist, which is commercialized by GSK.

As used herein, the term "DDR1/2" refers to Discoidin domain receptors 1 and 2. DDR1 and DDR2 constitute a unique subfamily of receptor tyrosine kinases and have been identified as nonintegrin receptors for collagens. DDR1 and DDR2 are differentiated from each other by their relative affinity for different types of collagens. DDR1 is activated by both fibrillar and nonfibrillar collagens, whereas DDR2 is activated only by fibrillar collagens, in particular, types I and III. As used herein, the term "inhibitor of DDR1/2" refers to a natural or synthetic compound that has a biological effect to inhibit the activity or the expression of DDR1/2. More particularly, such compound by inhibiting DDR1/2, blocks DDR1/2 activation or any of the downstream biological effects of DDR1/2 activation, reduces DDR1/2's kinase activity making the receptor inaccessible to their natural ligand.

In the present invention, the inhibitor of DDR1/2 is (4-(4-methylpiperazin-1-yhnethyl)-N-[4-methyl-3-(4-pyridin-3-yl) pyrimidin-2-ylamino)phenyl-benzamide) also known as STI571, Imatinib or GLIVEC^{®}; Novartis (International Patent Publication No. WO 95/09852).

In the last few years, staggering advances in sequencing technologies have provided an unprecedentedly detailed overview of the multiple genetic aberrations in cancer. By considerably expanding the list of new potential oncogenes and tumor suppressor genes, these new data strongly emphasize the need of fast and reliable strategies to characterize the normal and pathological function of these genes and assess their role, in particular as driving factors during oncogenesis. As an alternative to more conventional approaches, such as cDNA overexpression or downregulation by RNA interference, the new technologies provide the means to recreate the actual mutations observed in cancer through direct manipulation of the genome. Indeed, natural and engineered nuclease enzymes have attracted considerable attention in the recent years. The mechanism behind endonuclease-based genome inactivating generally requires a first step of DNA single or double strand break, which can then trigger two distinct cellular mechanisms for DNA repair, which can be exploited for DNA inactivating: the errorprone nonhomologous end-joining (NHEJ) and the high-fidelity homology-directed repair (HDR).

In another embodiment, the melanoma is resistant melanoma. As used herein, the term "resistant melanoma" refers to melanoma, which does not respond to a classical treatment. The cancer may be resistant at the beginning of treatment or it may become resistant during treatment. The resistance to drug leads to rapid progression of metastatic of melanoma. The resistance of cancer for the medication is caused by mutations in the gene, which are involved in the proliferation, divisions or differentiation of cells. In the context of the invention, the resistance of melanoma is caused by the mutations (single or double) in the following genes: BRAF, MEK, NRAS or PTEN. The resistance can be also caused by a double-negative BRAF and NRAS mutation. The NRAS gene is in the Ras family of oncogene and involved in regulating cell division. NRAS mutations in codons 12, 13, and 61 arise in 15-20 % of all melanomas. The inhibitors of BRAF mutation or MEK are used to treat the melanoma with NRAS mutations. In a particular embodiment, the melanoma is resistant in which double-negative BRAF and NRAS mutant melanoma. The term "PTEN" refers to Phosphatase and TENsin homolog, it is one of the most frequently inactivated tumor suppressor genes in sporadic cancers. Inactivating mutations and deletions of the PTEN gene are found in many types of cancers, including melanoma. The resistance can be also caused by a triple-negative NRAS, BRAF and MEK mutations as described above. Accordingly, such resistance is against to the treatments as described above.

As used herein, the term "classical treatment" refers to any compound, natural or synthetic, used for the treatment of a melanoma. In a particular embodiment, the classical treatment refers to radiation therapy, antibody therapy or chemotherapy.

As used herein, the term "chemotherapy" has its general meaning in the art and refers to chemical compounds that are effective in inhibiting tumor growth. Examples of chemotherapeutic agents include multkinase inhibitors such as sorafenib and sunitinib, alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33: 183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalamycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defo famine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK^{®}; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.].) and doxetaxel (TAXOTERE^{®}, Rhone-Poulenc Rorer, Antony, France); chlorambucil; gemcitabine; 6-thioguanine; mercaptopurine; methotrexate; platinum analogs such as cisp latin and carbop latin; vinblastine; platinum; etoposide (VP- 16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylomithine (DMFO); retinoic acid; capecitabine; and pharmaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

As used herein, the term "radiation therapy" has its general meaning in the art and refers the treatment of cancer with ionizing radiation. Ionizing radiation deposits energy that injures or destroys cells in the area being treated (the target tissue) by damaging their genetic material, making it impossible for these cells to continue to grow. One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons produce x-rays of increasingly greater energy. The use of machines to focus radiation (such as x-rays) on a cancer site is called external beam radiation therapy. Gamma rays are another form of photons used in radiation therapy. Gamma rays are produced spontaneously as certain elements (such as radium, uranium, and cobalt 60) release radiation as they decompose, or decay. In some embodiments, the radiation therapy is external radiation therapy. Examples of external radiation therapy include, but are not limited to, conventional external beam radiation therapy; three-dimensional conformal radiation therapy (3D-CRT), which delivers shaped beams to closely fit the shape of a tumor from different directions; intensity modulated radiation therapy (IMRT), e.g., helical tomotherapy, which shapes the radiation beams to closely fit the shape of a tumor and also alters the radiation dose according to the shape of the tumor; conformal proton beam radiation therapy; image-guided radiation therapy (IGRT), which combines scanning and radiation technologies to provide real time images of a tumor to guide the radiation treatment; intraoperative radiation therapy (IORT), which delivers radiation directly to a tumor during surgery; stereotactic radiosurgery, which delivers a large, precise radiation dose to a small tumor area in a single session; hyperfractionated radiation therapy, e.g., continuous hyperfractionated accelerated radiation therapy (CHART), in which more than one treatment (fraction) of radiation therapy are given to a subject per day; and hypofractionated radiation therapy, in which larger doses of radiation therapy per fraction is given but fewer fractions.

In another embodiment, the melanoma is resistant to a combined treatment. As used herein, the terms "combined treatment", "combined therapy" or "therapy combination" refer to a treatment that uses more than one medication. The combined therapy may be dual therapy or bi-therapy. In the context of the invention, the melanoma is resistant to a combined treatment characterized by using an inhibitor of BRAF mutation and an inhibitor of MEK as described above. For example, the combined treatment may be a combination of Vemurafenib and Cotellic. In a further embodiment, the melanoma is resistant to a treatment with the inhibitors of MEK.

In a further embodiment, the melanoma is resistant to a treatment with an immune checkpoint inhibitor.

As used herein, the term "immune checkpoint inhibitor" refers to molecules that totally or partially reduce, inhibit, interfere with or modulate one or more immune checkpoint proteins. As used herein, the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al., 2011. Nature 480:480- 489). Examples of stimulatory checkpoint include CD27 CD28 CD40, CD122, CD137, OX40, GITR, and ICOS. Examples of inhibitory checkpoint molecules include A2AR, B7-H3, B7-H4, BTLA, CTLA-4, CD277, IDO, KIR, PD-1, LAG-3, TIM-3 and VISTA. The Adenosine A2A receptor (A2AR) is regarded as an important checkpoint in cancer therapy because adenosine in the immune microenvironment, leading to the activation of the A2a receptor, is negative immune feedback loop and the tumor microenvironment has relatively high concentrations of adenosine. B7-H3, also called CD276, was originally understood to be a co-stimulatory molecule but is now regarded as co-inhibitory. B7-H4, also called VTCN1, is expressed by tumor cells and tumor-associated macrophages and plays a role in tumour escape. B and T Lymphocyte Attenuator (BTLA) and also called CD272, has HVEM (Herpesvirus Entry Mediator) as its ligand. Surface expression of BTLA is gradually downregulated during differentiation of human CD8+ T cells from the naive to effector cell phenotype, however tumor-specific human CD8+ T cells express high levels of BTLA. CTLA-4, Cytotoxic T-Lymphocyte-Associated protein 4 and also called CD152. Expression of CTLA-4 on Treg cells serves to control T cell proliferation. IDO, Indoleamine 2,3-dioxygenase, is a tryptophan catabolic enzyme. A related immune-inhibitory enzymes. Another important molecule is TDO, tryptophan 2,3-dioxygenase. IDO is known to suppress T and NK cells, generate and activate Tregs and myeloid-derived suppressor cells, and promote tumour angiogenesis. KIR, Killer-cell Immunoglobulin-like Receptor, is a receptor for MHC Class I molecules on Natural Killer cells. LAG3, Lymphocyte Activation Gene-3, works to suppress an immune response by action to Tregs as well as direct effects on CD8+ T cells. PD-1, Programmed Death 1 (PD-1) receptor, has two ligands, PD-L1 and PD-L2. This checkpoint is the target of Merck & Co.'s melanoma drug Keytruda, which gained FDA approval in September 2014. An advantage of targeting PD-1 is that it can restore immune function in the tumor microenvironment. TIM-3, short for T-cell Immunoglobulin domain and Mucin domain 3, expresses on activated human CD4+ T cells and regulates Th1 and Th17 cytokines. TIM-3 acts as a negative regulator of Th1/Tc1 function by triggering cell death upon interaction with its ligand, galectin-9. VISTA, Short for V-domain Ig suppressor of T cell activation, VISTA is primarily expressed on hematopoietic cells so that consistent expression of VISTA on leukocytes within tumors may allow VISTA blockade to be effective across a broad range of solid tumors. Tumor cells often take advantage of these checkpoints to escape detection by the immune system. Thus, inhibiting a checkpoint protein on the immune system may enhance the anti-tumor T-cell response.

In some embodiments, an immune checkpoint inhibitor refers to any compound inhibiting the function of an immune checkpoint protein. Inhibition includes reduction of function and full blockade. In some embodiments, the immune checkpoint inhibitor could be an antibody, synthetic or native sequence peptides, small molecules or aptamers which bind to the immune checkpoint proteins and their ligands.

In a particular embodiment, the immune checkpoint inhibitor is an antibody.

Typically, antibodies are directed against A2AR, B7-H3, B7-H4, BTLA, CTLA-4, CD277, IDO, KIR, PD-1, LAG-3, TIM-3 or VISTA.

In a particular embodiment, the immune checkpoint inhibitor is an anti-PD-1 antibody such as described in WO2011082400, WO2006121168, WO2015035606, WO2004056875, WO2010036959, WO2009114335, WO2010089411, WO2008156712, WO2011110621, WO2014055648 and WO2014194302. Examples of anti-PD-1 antibodies which are commercialized: Nivolumab (Opdivo^{®}, BMS), Pembrolizumab (also called Lambrolizumab, KEYTRUDA^{®} or MK-3475, MERCK).

In some embodiments, the immune checkpoint inhibitor is an anti-PD-L1 antibody such as described in WO2013079174, WO2010077634, WO2004004771, WO2014195852, WO2010036959, WO2011066389, WO2007005874, WO2015048520, US8617546 and WO2014055897. Examples of anti-PD-L1 antibodies which are on clinical trial: Atezolizumab (MPDL3280A, Genentech/Roche), Durvalumab (AZD9291, AstraZeneca), Avelumab (also known as MSB0010718C, Merck) and BMS-936559 (BMS).

In some embodiments, the immune checkpoint inhibitor is an anti-PD-L2 antibody such as described in US7709214, US7432059 and US8552154.

In the context of the invention, the immune checkpoint inhibitor inhibits Tim-3 or its ligand.

In a particular embodiment, the immune checkpoint inhibitor is an anti-Tim-3 antibody such as described in WO03063792, WO2011155607, WO2015117002, WO2010117057 and WO2013006490.

In some embodiments, the immune checkpoint inhibitor is a small organic molecule.

The term "small organic molecule" as used herein, refers to a molecule of a size comparable to those organic molecules generally used in pharmaceuticals. The term excludes biological macro molecules (e. g. proteins, nucleic acids, etc.). Typically, small organic molecules range in size up to about 5000 Da, more preferably up to 2000 Da, and most preferably up to about 1000 Da.

Typically, the small organic molecules interfere with transduction pathway of A2AR, B7-H3, B7-H4, BTLA, CTLA-4, CD277, IDO, KIR, PD-1, LAG-3, TIM-3 or VISTA.

In a particular embodiment, small organic molecules interfere with transduction pathway of PD-1 and Tim-3. For example, they can interfere with molecules, receptors or enzymes involved in PD-1 and Tim-3 pathway.

In a particular embodiment, the small organic molecules interfere with Indoleamine-pyrrole 2,3-dioxygenase (IDO) inhibitor. IDO is involved in the tryptophan catabolism (Liu et al 2010, Vacchelli et al 2014, Zhai et al 2015). Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6-fluoro-tryptophan, 4-methyl-tryptophan, 5 -methyl tryptophan, 6-methyl-tryptophan, 5-methoxy-tryptophan, 5 -hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a β-carboline derivative or a brassilexin derivative. In a particular embodiment, the IDO inhibitor is selected from 1-methyl-tryptophan, β-(3- benzofuranyl)-alanine, 6-nitro-L-tryptophan, 3-Amino-naphtoic acid and β-[3- benzo(b)thienyl] -alanine or a derivative or prodrug thereof.

In a particular embodiment, the inhibitor of IDO is Epacadostat, (INCB24360, INCB024360) has the following chemical formula in the art and refers to -N-(3-bromo-4-fluorophényl)-N'-hydroxy-4-{[2-(sulfamoylamino)-éthyl]amino}-1,2,5-oxadiazole-3 carboximidamide :

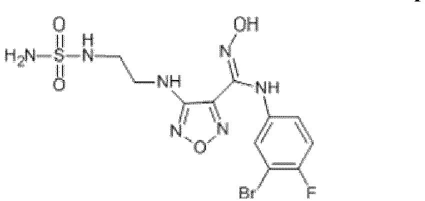

In a particular embodiment, the inhibitor is BGB324, also called R428, such as described in WO2009054864, refers to 1H-1,2,4-Triazole-3,5-diamine, 1-(6,7-dihydro-5H-benzo[6,7]cyclohepta[1,2-c]pyridazin-3-yl)-N3-[(7S)-6,7,8,9-tetrahydro-7-(1-pyrrolidinyl)-5H-benzocyclohepten-2-yl]- and has the following formula in the art:

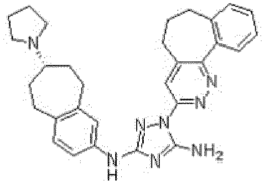

In a particular embodiment, the inhibitor is CA-170 (or AUPM-170): an oral, small molecule immune checkpoint antagonist targeting programmed death ligand-1 (PD-L1) and V-domain Ig suppressor of T cell activation (VISTA) (Liu et al 2015). Preclinical data of CA-170 are presented by Curis Collaborator and Aurigene on November at ACR-NCI-EORTC International Conference on Molecular Targets and Cancer Therapeutics.

In some embodiments, the immune checkpoint inhibitor is an aptamer.

Typically, the aptamers are directed against A2AR, B7-H3, B7-H4, BTLA, CTLA-4, CD277, IDO, KIR, PD-1, LAG-3, TIM-3 or VISTA.

In a particular embodiment, aptamers are DNA aptamers such as described in Prodeus et al 2015. A major disadvantage of aptamers as therapeutic entities is their poor pharmacokinetic profiles, as these short DNA strands are rapidly removed from circulation due to renal filtration. Thus, aptamers according to the invention are conjugated to with high molecular weight polymers such as polyethylene glycol (PEG). In a particular embodiment, the aptamer is an anti-PD-1 aptamer. Particularly, the anti-PD-1 aptamer is MP7 pegylated as described in Prodeus et al 2015.

As used herein the terms "administering" or "administration" refer to the act of injecting or otherwise physically delivering a substance as it exists outside the body (e.g., an inhibitor of BRAF, MEK and DDR1/2) into the subject, such as by mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

In a particular embodiment, i)the inhibitor of BRAF, ii) the inhibitor of MEK and iii) the inhibitor of DDR1/2 are administered to the subject in need thereof simultaneously, separately or sequentially.

As used herein, the term "administration simultaneously" refers to administration of 2 active ingredients by the same route and at the same time or at substantially the same time. The term "administration separately" refers to an administration of 2 active ingredients at the same time or at substantially the same time by different routes. The term "administration sequentially" refers to an administration of 2 active ingredients at different times, the administration route being identical or different.

By a "therapeutically effective amount" is meant a sufficient amount of inhibitor of BRAF, MEK and DDR1/2 for use in a method for the treatment of melanoma at a reasonable benefit/risk ratio applicable to any medical treatment. It will be understood that the total daily usage of the compounds and compositions of the present invention will be decided by the attending physician within the scope of sound medical judgment. The specific therapeutically effective dose level for any particular subject will depend upon a variety of factors including the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, typically from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The present invention also relates to a pharmaceutical composition comprising i) vemurafenib, an inhibitor of BRAF, ii) trametinib, an inhibitor of MEK and iii) imatinib, an inhibitor of DDR1/2.

In a particular embodiment, the invention refers to a pharmaceutical composition comprising i) vemurafenib, an inhibitor of BRAF, ii) trametinib, an inhibitor of MEK and iii) imatinib, an inhibitor of DDR1/2, for use in the therapy.

In a particular embodiment, the invention refers to a pharmaceutical composition comprising i) vemurafenib, an inhibitor of BRAF, ii) trametinib, an inhibitor of MEK and iii) imatinib, an inhibitor of DDR1/2, for use in the treatment of melanoma.

In one embodiment, the pharmaceutical composition of the invention is administered in combination with a classical treatment of melanoma.

In particular embodiment, the pharmaceutical composition of the invention is administered in combination with an anti-PD-1 antibody.

The inhibitors of BRAF, MEK and/or DDR1/2 as described above may be combined with pharmaceutically acceptable excipients, and sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The invention will be further illustrated by the following figures and examples. However, these examples and figures should not be interpreted in any way as limiting the scope of the present invention.

### FIGURES:

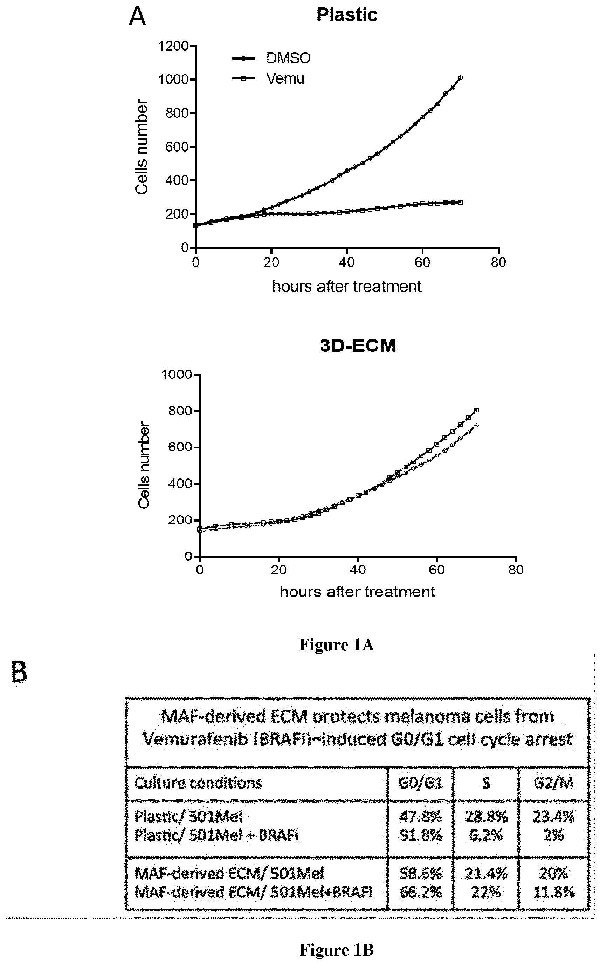

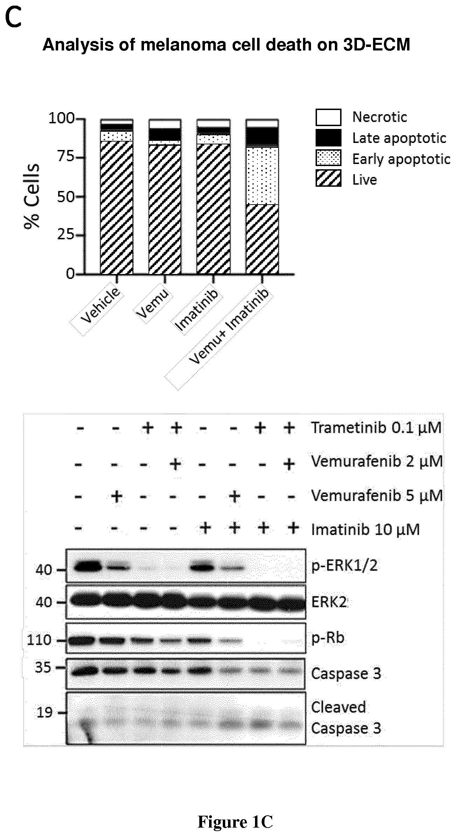

**Figure 1**. (A) Fibroblast-derived 3D ECM confers drug-protective action to **melanoma cells against anti-BRAFV600E therapies.** Proliferation of 501Mel cells plated on plastic or on decellularized 3D cell-derived matrices generated from melanoma-associated fibroblasts (MAF) for the indicated times. After 2 days, cells were treated with vehicle (DMSO) or 2 µM Vemurafenib (BRAFi). Time-lapse analysis of fluorescently NucLightTM-labeled cells using the IncuCyte ZOOM system. **(B) Cell cycle distribution of 501Mel cells cultured on plastic or MAF-derived ECM for 48 h and treated for an additional 48 h with 2 µM vemurafenib (BRAFi).** Cell cycle profiles were analyzed by flow cytometry of PI-stained cells. The percentage of cells in different phases of the cell cycle is indicated. **(C) Inhibition of DDR1/2 by Imatinib overcomes ECM-mediated resistance to combined BRAF and MEK inhibition, and leads to apoptotic cell death.** *Upper panel,* 1205Lu melanoma cells cultured on MAF-derived ECM for 48 h were treated for an additional 48 h with 2 µM Vemurafenib (BRAFi), 10 µM Imatinib (DDR1/2i) alone or a combination of both. Apoptosis was analysed by flow cytometry after labelling of cells with AnnexinV-FITC and propidium iodide (PI). The percentage of different forms of cell death based on Annexin-FITC and PI positivity is indicated. *Lower panel,* Western blot analysis of phosphorylated form of Rb and Caspase 3 cleavage in 1205Lu cells cultured on 3D-ECM and treated with Vemurafenib (BRAFi), Trametinib (MEKi), Imatinib (DDR1/2i) alone or in combination as indicated.

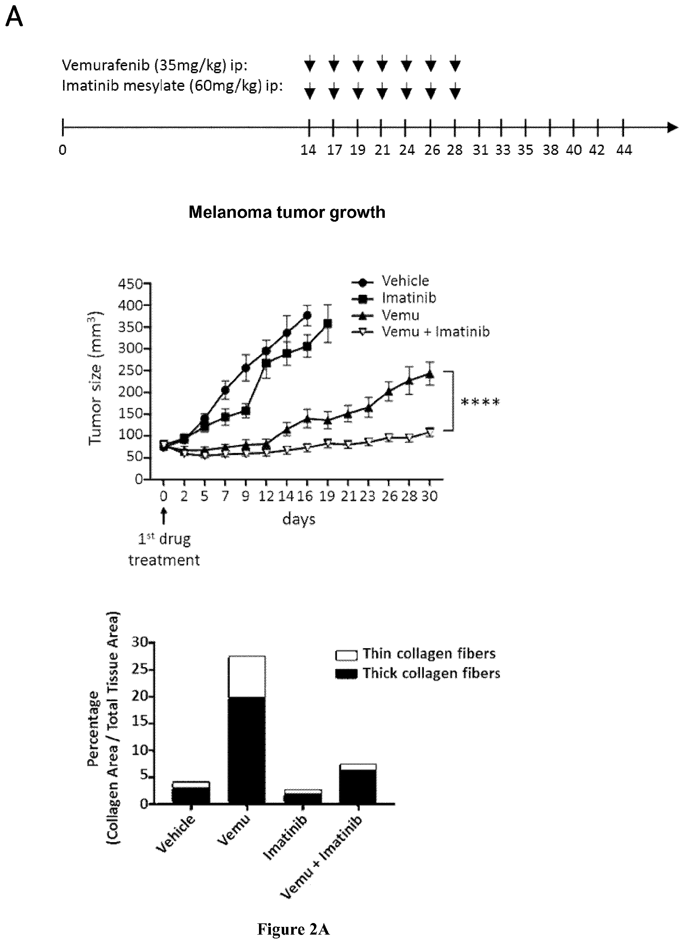

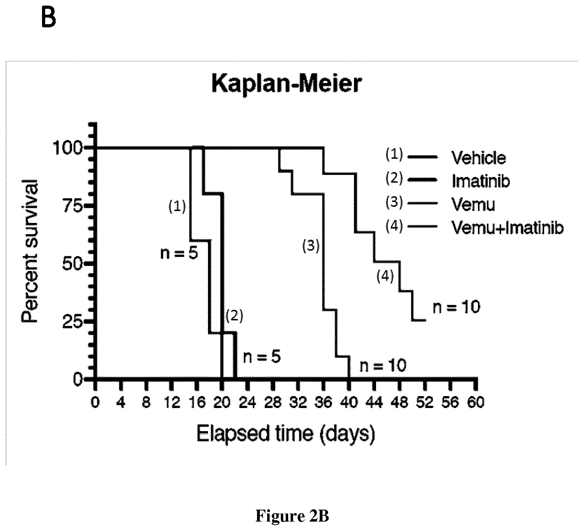

**Figure 2**. (A) Targeting DDR1 and DDR2 collagen receptors by Imatinib sensitizes **melanoma tumors to BRAFV600E targeted therapy and normalizes the fibrotic stromal reaction.** 1205Lu cells (1 × 10⁶) were injected sc into nude mice. Tumor volume was assessed with caliper and when tumors reached 100 mm3, mice were randomized into four groups and treated at the indicated date with vehicle or ip injections of Vemurafenib (BRAFi) (35 mg/kg), Imatinib mesylate (60 mg/kg), or Vemurafenib plus Imatinib for 30 days. Graphs show tumor growth following treatment by indicated drugs (mono or combo therapy). Data shown are mean ± SEM of tumor volume (n = 6; ****p<0.0001). Two-way ANOVA followed by Tukey's multiple comparisons test was used for statistical analyses. Sections of 1205Lu xenografts were stained with picrosirius red and imaged under polarized light microscopy to ascertain collagen maturity and fiber thickness. Birefringence hue and amount of collagen fibers were quantified as a percent of total tissue area. **(B) Kaplan-Meier survival curves of 1205Lu melanoma-bearing mice treated with Vehicle (n=5), Imatinib mesylate alone (n=5), Vemurafenib alone (n=10) and Vemurafenib + Imatinib mesylate (n=10).** Median time to progression was 18, 20, 36 and 48 days, respectively. Log rank (Mantel-Cox) for Vemurafenib versus Vmurafenib + Imatinib mesylate: ***p<0.0001 and hazard ration (log rank): 0,2403 (95% Cl of ration, 0,08123 to 0,7106).

### EXAMPLE:

### Material & Methods

### Cell and reagents

Melanoma cells and MAFs were cultured in Dulbecco's modified Eagle Medium (DMEM) plus 7% FBS (Hyclone). Culture reagents were purchased from Thermo Fisher Scientific. All other reagents were purchased from Sigma-Aldrich unless stated otherwise. PLX4032 (Vemurafenib), GSK1120212 (Trametinib), RO5126766, Imatinib mesylate, Nilotinib were from Selleckem. Collagen I rat tail was from Thermo Fisher Scientific.

### RNAi studies

Non-targeting control, DDR1 and DDR2 siRNA duplexes were designed by Invitrogen (Thermo Fisher Scientific). Transfection of siRNA was carried out using Lipofectamine RNAiMAX (Thermo Fisher Scientific), at a final concentration of 50 nM.

### Cell-derived Extracellular matrix (ECM) preparation and MM-DR assay

Gelatin-coated tissue culture dishes were seeded with fibroblasts and cultured for 8 days in complete medium, supplemented with 50 µg/ml ascorbic acid every 48 h. Cell cultures were then washed with PBS and matrices were denuded following a 2 min treatment with prewarmed extraction buffer (PBS 0.5% Triton X-100, 20 µMNH4OH). Matrices were then gently washed several times with PBS. For the drug-protection assay (MM-DR), melanoma cells were seeded onto the decellularized 3D matrices for 48 h at 37°C in 5% CO2, and then cultivated in complete medium for another 48 h period in presence or not of the indicated amounts of targeted agents. Cells were detached and either fixed in 80% ethanol for cell cycle analysis or in RIPA buffer for immunoblot analysis.

### Cell proliferation assay

For realtime analysis of cell growth using the IncuCyteTM Zoom imaging system (Essen Bioscience), cells stably expressing the nuclear-restricted non-perturbing fluorescent label (red IncuCyte^{®} NucLight Reagent) were plated in triplicate in complete medium on 96-well plates (5 x 103cells/well) coated or not with fibroblast-derived ECM. Phase contrast images were taken every hour over a 3-day period. Cell proliferation is quantified by counting the number of fluorescent nuclei over time to give cell growth rates. Growth curves were generated using the IncuCyteTM cell proliferation assay software.

### Flow cytometry analysis

Cell cycle profiles were determined by flow cytometry analysis of propidium iodide (PI)-stained cells. Melanoma cells cultured onto cell-derived matrices and treated with or without 2 µM vemurafenib for 48 h were washed, fixed in 80% ethanol and incubated at -20°C for 24 h. Cells were then stained for 20 min at 37°C in buffer containing 40 µg/ml PI and 20 µg/ml ribonuclease A from bovine pancreas. Cell cycle profiles were collected on a FACS Canto. Cell death was evaluated by flow cytometry following staining with AnnexinV/PI (eBioscience). Melanoma cells cultured onto cell-derived matrices were double stained with AnnexinV-FITC and PI and analysed using a BD FACSCanto cytometer.

### Immunoblot analysis and antibodies

Melanoma cells cultured onto cell-derived matrices were lysed in RIPA buffer supplemented with protease and phosphatase inhibitors. Whole cell lysates were subjected to SDS-PAGE and immunoblot analysis. The following antibodies were used at working dilution of 1:1,000, unless otherwise stated. Anti ERK1/2, HSP90, HSP60 and DDR1 antibodies were from Santa Cruz Biotechnology. Anti DDR2, phospho-DDR1 (Tyr792), phospho-MEK1/2 (Ser217/221), phospho-ERK1/2 (T202/Y204), phospho-Rb (Ser807/811), p27KIP1, Survivin, BAX, BIM and Caspase 3 antibodies were from Cell Signaling Technology. Anti phospho-DDR2 (Tyr740) antibodies were from Bio-Techne. Peroxydase-conjugated anti-mouse and anti-rabbit antibodies were from Cell Signaling Technology. Peroxydase-conjugated anti-goat antibody was from Dako. Secondary antibodies were used at 1:3,000 dilution. Proteins were detected by enhanced chemiluminescence (ECL) Amersham Western Blotting Detection Reagents (GE Healthcare Life Sciences).

### Cell line-derived xenograft (CDX) tumor models

Mouse experiments were carried out in accordance with the Institutional Animal Care and the local ethical committee. 1 × 10⁶ 1205Lu melanoma cells were subcutaneously implanted into both flanks of 6 week old female athymic nude nu/nu mice (Envigo). Tumor was measured by caliper and the volume was calculated using the formula: V = tumor width × tumor length2 × 0.5. When tumor reached 100 mm³, mice were randomly grouped into control and test groups. Vemurafenib (35 mg/kg) and Imatinib mesylate were delivered (alone or in combination) intraperitoneally three times per week. Mice in control group were treated with vehicle. Mice were treated for 14 days and followed for up to 40 days or until tumors reached a pre-defined volume (1000 mm³). After animal sacrifice tumors were dissected, fixed in formalin and embedded in paraffin.

### Histopathological evaluation of collagen fibers

Paraffin-embedded xenograft tissues were cut into 10-µm sections and stained with Picrosirius red using standard protocols. Picrosirius red stained sections were analyzed under Polarizing Microscope to analyze the nature of collagen (thickness, organization, hue, density, and birefringence).

### Results

To investigate the contribution of ECM components and of collagen signaling through DDR, in MM-DR, inventors generated 3D-ECM from MAFs and tested their effectiveness to protect BRAFV600E-mutated melanoma cells (501Mel or 1205Lu cells) against the anti-proliferative effects of inhibition of the BRAF oncogenic pathway. Cell-derived matrices were generated, denuded of cells and their architecture, topology and molecular composition were analyzed using proteomic and microscopic approaches. In their experimental conditions, MAFs produce and assemble *in vitro* a rich and complex ECM composed of Collagen and Fibronectin fibers that recapitulates many biochemical and biophysical properties of *in vivo* ECM (data not shown). 501Mel cells stably expressing a fluorescent nuclear label were cultured on matrices derived from MAF population isolated from melanoma metastatic biopsies, and treated or not with vemurafenib (BRAFi). Cell proliferation was monitored using live cell time-lapse *imaging* and quantified by counting the number of *fluorescent* nuclei. Cell growth inhibition induced by BRAFi was totally abrogated when cells are cultured on cell-derived ECM **(****Figure 1A****).** Cell cycle analysis showed that experimentally produced ECM prevented the G0/G1 cell cycle arrest induced by the targeted therapy in contrast to standard cell culture conditions on plastic **(****Figure 1B****).** Thus, ECM transmits signals that prevent the anti-proliferative action of BRAF inhibition. At the molecular level, matrix-mediated therapeutic escape from BRAF inhibition was associated in both 501Mel and 1205Lu cells with sustained levels of the proliferation marker CyclinD1, low levels of the cell cycle inhibitor p27KIP1 together with maintained phosphorylation of ERK1/2 in presence of the drug. Similar results were obtained with ECM generated from another MAF population (data not shown). Because clinical management of BRAF mutated melanomas currently involves combinatorial therapy integrating BRAF and MEK inhibitors, they next tested whether cell-derived ECM could be effective in protecting drug-sensitive melanoma cells from BRAF and MEK co-inhibition. Matrices generated by MAF also significantly prevented the effect of dual inhibition on 501Mel cells observed on plastic conditions, evidenced by maintaining phosphorylation of the proliferation marker Rb, low levels of p27KIP1 and expression level of the anti-apoptotic protein Survivin (data not shown). Upregulation of expression levels of the pro-apoptotic protein BIM observed in response to the drugs was also prevented after exposure of cells to cell-derived ECM. Note that expression levels of BAX, another pro-apoptotic protein, remained unchanged in the different experimental settings analyzed (data not shown).

These findings demonstrate that melanoma cell lines were protected by the experimentally derived MAF matrix against the growth and survival inhibitory effects induced by MAPK targeting drugs compared with standard growth conditions on plastic. These results thus indicate a critical role for the ECM in creating a permissive niche for resistance to targeted therapies in melanoma.

The functional contribution of DDR1 and DDR2 signaling pathway to matrix-mediated resistance to BRAF inhibition was next explored. Immunoblot analysis of 1205Lu melanoma cells stimulated with collagen I revealed a slow but persistent increase in DDR1 and DDR2 tyrosine kinase activities, as measured by DDR1-Tyr792 and DDR2 Tyr740 autophosphorylation (data not shown). To address the contribution of the two receptors for collagens in MM-DR, a siRNA approach was used to target DDR1, DDR2 or both in 1205Lu cells. Immunoblot analysis showed specific protein reduction after siRNA transfection in single target and target combination treatments in 1205Lu cells cultured on MAF-derived ECM (data not shown). Compared to the single knockdown, the simultaneous knockdown of DDR1 and DDR2 overcame MM-DR to BRAF targeted therapy as revealed by decreased levels of phospho-Rb and Survivin. As DDRs are tyrosine kinase receptors and druggable targets, inventors used Imatinib to inhibit DDR kinase activities in the drug-protection assay. Imatinib is a tyrosine kinase inhibitor (TKI) developed as a BCR-ABL kinase inhibitor but later shown to inhibit DDR1 and DDR2 kinase activities highly efficiently. Imatinib belongs to therapeutic molecules used in the clinic for the treatment of Chronic Myeloid Leukemia (CML). Nilotinib, another BCR-ABL inhibitor has been developed later and approved for the treatment of patients with resistance to Imatinib. They first confirmed that Imatinib efficiently abrogated type I collagen-mediated DDR1 and DDR2 tyrosine phosphorylation in melanoma cells (data not shown). Inhibition of DDR1 and DDR2 kinase activities by Imatinib suppressed the protection of melanoma cells from Vemurafenib (BRAFi) and Trametinib (MEKi) co-drugging and led to reduced Rb phosphorylation and cleavage of Caspase 3, a sign of apoptotic cell death **(****Figure 1C****).** Induction of apoptosis caused by Imatinib in presence of Vemurafenib was further confirmed on 1205Lu melanoma cells cultured on 3D-ECM by cytometry analysis **(****Figure 1C****).** Similar biochemical cell cycle and apoptotic events were promoted in presence of Nilotinib (data not shown).

This indicates that DDR1 and DDR2 determine BRAF mutant melanoma cells responsiveness to targeted therapies and that the drug-protective action of DDRs is dependent of their enzymatic activities.

Finally, the anti-tumor activity of Imatinib combined with Vemurafenib was validated in a pre-clinical xenograft model of melanoma. BRAF-mutated melanoma cells 1205Lu were subcutaneously xenografted into nude mice (CDX model), which were exposed to Vemurafenib, Imatinib or Vemurafenib plus Imatinib **(****Figure 2****).** As expected, BRAF targeting induced a rapid inhibition of tumor growth, whereas Imatinib did not display any significant anti-melanoma effect. However, after 12 days, tumors treated with BRAFi alone had resumed growth, whereas combination with Imatinib markedly delayed the development of resistance and led to a significant reduction of tumor growth. Histochemical analysis of Collagen content and organization showed that Vemurafenib treatment triggered a profound remodeling of the melanoma ECM stroma, with marked increase of Collagen fibers thickness that was suppressed by Imatinib **(****figure 2A****).** This data suggests that treatment with Imatinib counteracts the adverse effect of targeted agents on aberrant Collagen deposition, a process potentially contributing to drug resistance and relapse. Moreover, the Vemurafenib/Imatinib combination significantly increased the survival of mice that were treated for 14 days **(****figure 2B****)** without apparent weight loss or signs of organ toxicity (data not shown).

From these observations, inventors conclude that targeting DDR1 and DDR2 collagen receptors by Imatinib sensitizes melanoma tumors to BRAFV600E targeted therapy, prevents tumor relapse seen with BRAFi alone, and normalizes the fibrotic stromal reaction. These findings provide the rationale to combine Imatinib (or other DDR inhibitors) and MAPK-targeting agents to disrupt the influence of the matrix microenvironment in order to delay or prevent the emergence of therapy-resistant cells.

### REFERENCES:

Throughout this application, various references describe the state of the art to which this invention pertains.

## Claims

1. A therapeutically effective amount of: i) vemurafenib, an inhibitor of BRAF, ii) trametinib, an inhibitor of MEK and iii) imatinib, an inhibitor of DDR1/2 for use in the treatment of melanoma in a subject in need thereof.

2. The therapeutically effective amount of: i) the vemurafenib, inhibitor of BRAF, ii) the trametinib, inhibitor of MEK and iii) the imatinib, inhibitor of DDR1/2 for use according to claim 1, wherein the melanoma is resistant melanoma.

3. The therapeutically effective amount of: i) the vemurafenib, inhibitor of BRAF, ii) the trametinib, inhibitor of MEK and iii) the imatinib, inhibitor of DDR1/2 for use according to claim 1, wherein, i) the vemurafenib, ii) the trametinib and iii) the imatinib are administered in combination with a classical treatment of melanoma.

4. The therapeutically effective amount of: i) the vemurafenib, inhibitor of BRAF, ii) the trametinib, inhibitor of MEK and iii) the imatinib, inhibitor of DDR1/2 for use according to claim 1, wherein, i) the vemurafenib, ii) the trametinib and iii) the imatinib are administered in combination with an anti-PD-1 antibody.

5. A pharmaceutical composition comprising i) the vemurafenib, inhibitor of BRAF, ii) the trametinib, inhibitor of MEK and iii) the imatinib, inhibitor of DDR1/2.

6. The pharmaceutical composition according to claim 5, for use in the therapy.

7. The pharmaceutical composition according to claim 5, for use in the treatment of melanoma.

## Patentansprüche

1. Therapeutisch wirksame Menge von: i) Vemurafenib, einem BRAF-Inhibitor, ii) Trametinib, einem MEK-Inhibitor und iii) Imatinib, einem DDR 1/2-Inhibitor zur Verwendung bei der Behandlung von Melanom in einem Subjekt, das diese benötigt.

2. Therapeutisch wirksame Menge von: i) dem Vemurafenib, BRAF-Inhibitor, ii) dem Trametinib, MEK-Inhibitor und iii) dem Imatinib, DDR 1/2-Inhibitor zur Verwendung nach Anspruch 1, wobei das Melanom resistentes Melanom ist.

3. Therapeutisch wirksame Menge von: i) dem Vemurafenib, BRAF-Inhibitor, ii) dem Trametinib, MEK-Inhibitor und iii) dem Imatinib, DDR 1/2-Inhibitor zur Verwendung nach Anspruch 1, wobei i) das Vemurafenib, ii) das Trametinib und iii) das Imatinib in Kombination mit einer klassischen Melanom-Behandlung verabreicht werden.

4. Therapeutisch wirksame Menge von: i) dem Vemurafenib, BRAF-Inhibitor, ii) dem Trametinib, MEK-Inhibitor und iii) dem Imatinib, DDR 1/2-Inhibitor zur Verwendung nach Anspruch 1, wobei i) das Vemurafenib, ii) das Trametinib und iii) das Imatinib in Kombination mit einem PD-1-Antikörper verabreicht werden.

5. Pharmazeutische Zusammensetzung, umfassend i) das Vemurafenib, BRAF-Inhibitor, ii) das Trametinib, MEK-Inhibitor und iii) das Imatinib, DDR 1/2-Inhibitor.

6. Pharmazeutische Zusammensetzung nach Anspruch 5 zur Verwendung in der Therapie.

7. Pharmazeutische Zusammensetzung nach Anspruch 5 zur Verwendung bei der Behandlung von Melanom.

## Revendications

1. Quantité thérapeutiquement efficace de : i) vemurafenib, un inhibiteur de BRAF, ii) trametinib, un inhibiteur de MEK et iii) imatinib, un inhibiteur de DDR1/2 pour usage dans le traitement de mélanome chez un sujet en ayant besoin.

2. Quantité thérapeutiquement efficace de : i) vemurafenib, un inhibiteur de BRAF, ii) trametinib, un inhibiteur de MEK et iii) imatinib, un inhibiteur de DDR1/2 pour usage selon la revendication 1, dans laquelle le mélanome est un mélanome résistant.

3. Quantité thérapeutiquement efficace de : i) vemurafenib, un inhibiteur de BRAF, ii) trametinib, un inhibiteur de MEK et iii) imatinib, un inhibiteur de DDR1/2 pour usage selon la revendication 1, dans laquelle i) vemurafenib, ii) trametinib et iii) imatinib sont administrés en combinaison avec un traitement classique de mélanome.

4. Quantité thérapeutiquement efficace de : i) vemurafenib, un inhibiteur de BRAF, ii) trametinib, un inhibiteur de MEK et iii) imatinib, un inhibiteur de DDR1/2 pour usage selon la revendication 1, dans laquelle i) vemurafenib, ii) trametinib et iii) imatinib sont administrés en combinaison avec un anticorps anti-PD-1.

5. Composition pharmaceutique comprenant i) vemurafenib, un inhibiteur de BRAF, ii) trametinib, un inhibiteur de MEK et iii) imatinib, un inhibiteur de DDR1/2.

6. Composition pharmaceutique selon la revendication 5, pour usage thérapeutique.

7. Composition pharmaceutique selon la revendication 5, pour usage dans le traitement de mélanome.
